(19) Europäisches Patentamt
European Patent Office
Office européen des brevets

(11) Publication number: **0 136 280 A2**

# (12) EUROPEAN PATENT APPLICATION

(21) Application number: **84870132.2**

(22) Date of filing: **11.09.84**

(51) Int. Cl.[4]: **C 07 C 179/10**, C 07 C 178/00, C 11 D 3/39

(30) Priority: **12.09.83 US 531103**

(43) Date of publication of application: **03.04.85 Bulletin 85/14**

(84) Designated Contracting States: **AT BE CH DE FR GB IT LI LU NL SE**

(71) Applicant: **Monsanto Company, Patent Department 800 North Lindbergh Boulevard, St. Louis Missouri 63167 (US)**

(72) Inventor: **Alul, Husni Rushdi, 9677 Grandview Drive, St. Louis Missouri 63132 (US)**

(74) Representative: **Lunt, John Cooper et al, Monsanto Europe S.A. Patent Department Avenue de Tervuren 270-272 Letter Box No 1, B-1150 Brussels (BE)**

## (54) Production of substituted-butanediperoxoic acids.

(57) Substituted-butanediperoxoic acid is produced by reacting substituted succinic anhydride with a mixture of concentrated sulfuric acid and hydrogen peroxide. The substituted-butanediperoxoic acids are particularly useful as bleaching agents.

EP 0 136 280 A2

PRODUCTION OF SUBSTITUTED-BUTANEDIPEROXOIC ACIDS

This invention relates to a process for making substituted-butanediperoxoic acids. More particularly, it relates to the production of substituted butanediperoxoic acid by peroxidizing a substituted succinic anhydride in a sulfuric acid reaction medium.

BACKGROUND OF THE INVENTION

Diperoxoic acids are useful as bleaching agents, oxidizing agents, disinfecting agents, and the like.

Methods of preparing various classes of diperoxoic acid are known. U.S. Patent 2,377,038 issued May 29, 1945 to J. S. Reichert et al discloses a process for preparing organic peracid by reacting an anhydride of a carboxylic acid and an alkaline solution of an inorganic peroxygen compound in an aqueous medium at a pH of 8.0 - 11.0. U.S. Patent 2,813,896 issued November 19, 1957 to K. Krimm discloses a process for preparing organic peracids by reacting monocarboxylic and dicarboxylic aliphatic, cycloaliphatic and aromatic acids and acid anhydrides with aqueous hydrogen peroxide solution in sulfuric acid using such amounts that $H_2SO_4$ and $H_2O$ are at the rate of at least 1 mole to 6 moles at the end of the reaction. U.S. Patent 3,655,738 issued April 11, 1972 to D. R. Nelson discloses the preparation of diperphthalic acids by the reaction of phthalic acid in the form of finely divided particles having a dimension below 0.1 millimeter and hydrogen peroxide in an alkane sulfonic acid medium. U.S. Patent 4,233,235 issued November 11, 1980 to J. B. Camden et al discloses a process for making aliphatic diperoxyacids having 8 to 16 carbon atoms by continuously adding a dibasic acid of the formula $HOOC - (CH_2)_n - COOH$, sulfuric acid, hydrogen peroxide and water to a reactor and continuously withdrawing the diperoxyacid formed to maintain a constant residence

time for the reactants in the reactor. Similarly, U.S. Patent 4,244,884 issued January 13, 1981 to J. P. Hutchins et al relates to Camden et al and discloses a continuous process with two recycling loops.

U.S. Application Serial No. 336,542 filed on January 4, 1982 by J. M. Mayer, said application being owned by the owners of the present application, discloses substituted-butanediperoxoic acid bleaching agents. The preparation of the substituted butanediperoxoic acids shown in Serial No. 336,542 is by the addition of hydrogen peroxide to a stirred mixture of substituted succinic anhydride and methanesulfonic acid. Although this method of preparation using methanesulfonic acid as the reaction medium provides the desired substituted-butanediperoxoic acid, a more efficient process which can be employed on a commercial scale is highly desirable.

It has now been found that the substituted-butanediperoxoic acids can be produced in a more efficient manner using a concentrated sulfuric acid reaction medium which provides the advantages of improved reaction rates, less tedious product cleanup procedures and efficient formulating procedures using the product as an active agent for bleach formulations.

## SUMMARY OF THE INVENTION

These and other advantages of the present invention are achieved by the process for making substituted butanediperoxoic acids which comprises reacting a substituted succinic anhydride with a mixture of concentrated sulfuric acid and hydrogen peroxide.

The substituted-butanediperoxoic acids made in accordance with the present invention are particularly useful as bleaching agents as more fully described in U.S. Serial No. 336,542 filed on January 4, 1982 by J. M. Mayer, and can be represented by the general formula:

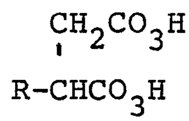

wherein R is alkyl of 6 to 18 carbon atoms or phenyl.

The substituted succinic anhydride employed in the present process can be represented by the general formula:

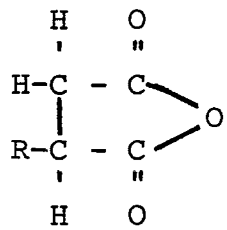

wherein R is alkyl of 6 to 18 carbon atoms or phenyl.

## DETAILED DESCRIPTION OF THE INVENTION

It has been found that substituted-butanediperoxoic acids can be prepared more efficiently in concentrated sulfuric acid reaction medium provided that the hydrogen peroxide reactant is mixed with the sulfuric acid reaction medium prior to contact with the substituted succinic anhydride reactant. In this manner, the formation of the corresponding substituted succinic acid is minimized and the reaction proceeds more rapidly to the formation of the substituted-butanediperoxoic acids. The substituted succinic anhydride reactants are available compounds and can be prepared by techniques known to those skilled in the art. For example, the alkyl substituted succinic anhydride can be prepared by the hydrogenation of alkenyl succinic anhydride prepared by the reaction of maleic anhydride with olefins. See, for example, U.S. Patent 3,412,111 issued November 19, 1968 to P. G. Irwin et al. Preferred substituted succinic anhydride reactants are $C_{8-12}$ alkyl substituted succinic anhydride wherein the alkyl substituent is, for example, octyl, decyl or dodecyl.

In the process of the present invention it is advantageous that the concentrated sulfuric acid

reaction medium employed be at least 60% $H_2SO_4$, preferably 75% to 98% $H_2SO_4$, more preferably 85% to 92% $H_2SO_4$. The substituted succinic anhydride reactant is soluble in this reaction medium and upon the formation of the substituted-butanediperoxoic acid it precipitates from the reaction medium in crystalline form.

The hydrogen peroxide reactant employed in the present process can be commercially available hydrogen peroxide of various concentrations such as 30%, 50% or 70% $H_2O_2$. Preferably, the higher concentrations, consistent with good safety and handling practices, are employed.

In carrying out the present invention the substituted succinic anhydride is reacted with a mixture of from about 10 moles to about 60 moles of sulfuric acid and from about 2 moles to about 18 moles of hydrogen peroxide per mole of the substituted succinic anhydride reactant. Preferably, the mixture contains from 20 moles to 40 moles $H_2SO_4$ and 4 moles to 12 moles $H_2O_2$ per mole of substituted succinic anhydride reactant.

While water is not separately added the concentration of the sulfuric acid and the hydrogen peroxide employed to provide the desired quantities in the mixture fixes the quantity of water in the mixture. Generally, the amount of water in the mixture will range from about 25 moles to about 125 moles per mole of the substituted succinic anhydride reactant. Employing the preferred higher concentrations of sulfuric acid and hydrogen peroxide improves the rate of reaction. Particularly advantageous results are achieved employing 85% to 92% $H_2SO_4$.

The present process can be conducted at temperatures that range from about 15°C or less up to about 60°C or more. Preferably, the reaction mass is maintained in the range of about 25° to about 55°C. The

reaction is exothermic and localized concentrations of the hydrogen peroxide reactant and the substituted succinic anhydride reactant are to be avoided for safety reasons.

Although the process can be conducted at below or above atmospheric pressure, it is advantageous to carry out the process at atmospheric pressure.

In the process of the present invention, the reaction can be carried out in a stirred conventional tank reactor. The interior surfaces of the reactor can be of any material which is non-reactive with the reactor contents. For example, it can be of ceramic, glass, Teflon, tantalum or zirconium. Teflon is a tradename for polytetrafluoroethylene polymers sold by the E. I. duPont de Nemours Company. Other materials for the interior surfaces of the reactor will suggest themselves to those skilled in the art. Interior reactor surfaces of metal which leave a residue in the products of reaction which later accelerate the decomposition of the substituted-butanediperoxoic acid reaction product, such as some stainless steels, are to be avoided. Generally, the reactor is provided with a cooling means in order to maintain the temperature of the reactor contents in the desired temperature range of 15°C to about 60°C, preferably 25° to 55°C. Suitable cooling means can be, for example, a chilled water jacket around the reactor.

The present process can be carried out batchwise or in a continuous manner. Preferably, the process is conducted by charging the substituted succinic anhydride to the stirred reactor containing a mixture of concentrated sulfuric acid and hydrogen peroxide to form a reaction mixture of from about 20 moles to 40 moles sulfuric acid and from about 4 moles to about 12 moles hydrogen peroxide per mole of substituted succinic anhydride. The stirred reaction

mass is maintained in a temperature range of about 15°C to about 60°C and allowed to react for a period from about 15 minutes to about 4 hours or more. Preferably, the reaction mass is maintained in the temperature range of about 25°C to about 55°C for about 45 minutes to about 150 minutes.

Upon charging the substituted succinic anhydride to the reactor containing the concentrated sulfuric acid and hydrogen peroxide mixture the substituted-butanediperoxoic acid reaction product begins to form and precipitates out of the reaction mixture forming a solid-liquid reaction mass. Upon substantial completion of the reaction, the reaction mass is filtered and the filter cake containing the substituted-butanediperoxoic acid reaction product is washed and dried. The liquid filtrate, that is, the mother liquor, containing sulfuric acid, hydrogen peroxide, water and small amounts of trapped reaction product or partially reacted product can be replenished with sufficient hydrogen peroxide and concentrated sulfuric acid as needed to provide the above described mole ratios and recycled back to the stirred reactor which is then charged with the substituted succinic anhydride reactant.

In a preferred embodiment, the present process is carried out in a continuous manner in which sulfuric acid of at least 60% concentration, preferably 85 to 92% $H_2SO_4$, is mixed with 30 - 70% hydrogen peroxide, preferably 50% $H_2O_2$ in sufficient quantities to provide a reaction medium containing from about 10 moles to about 60 moles $H_2SO_4$, preferably 20 moles to 40 moles $H_2SO_4$, and from about 2 moles to about 18 moles $H_2O_2$, preferably 4 moles to 12 moles $H_2O_2$ per mole of substituted succinic anhydride reactant. The higher mole ratios of $H_2SO_4$ and $H_2O_2$ are preferred for making $C_{12}$-$C_{18}$ substituted butanediperoxoic acids. Said

reaction medium is continuously fed to a stirred reactor system fitted with temperature control means while the substituted succinic anhydride reactant is added at such a rate to maintain the desired mole ratio. The reactor system can be a single vessel or a plurality of serially connected vessels each being so dimensioned and so equipped to provide sufficient mixing, temperature control and residence time of the reaction mass. The rate of introduction of reactants into and withdrawal from the reactor system, in comparison to the amount of material retained within the reactor system, is selected to give an effective residence time, preferably about 30 minutes to about 150 minutes. The reaction mass is maintained in the reactor system at a temperature in the range of about $15^{o}$ to $60^{o}C$ or more, preferably $25^{o}$ - $55^{o}C$, while the reaction mass is continuously withdrawn from the reactor system and the substituted-butanediperoxoic acid product is recovered by filtration or the like. The filtrate is replenished with sufficient hydrogen peroxide and concentrated sulfuric acid as needed and continuously recycled to the stirred reactor system.

The invention is further illustrated by, but not limited to, the following examples wherein all parts are by weight, unless otherwise indicated.

EXAMPLE 1

Twenty ml of 50% $H_2O_2$ (0.35 mole) were added to 200 grams of 75% sulfuric acid (1.5 moles) in a 250 ml beaker fitted with a stirrer at 0 - $10^{o}C$ and the mixture was heated to $50^{o}C$. Ten grams of n-octyl succinic anhydride (0.048 mole) were added and the mixture was stirred for one hour. The mixture was cooled to ambient temperature and the precipitated product was collected by filtering the mixture using a glass sintered funnel. The precipitated product was washed with deionized water until free from acid and air

dried. The recovered octylbutanediperoxoic acid was 10.6 grams (86% of theory). The product was analyzed for active oxygen by dissolving 50 mg in 0.5 ml of acetone, adding 1 ml of glacial acetic acid, followed by 10 ml of 5% KI and titrating with standard 0.1 N sodium thiosulfate solution. The sample showed 12.0% active oxygen (98.4% of theory).

EXAMPLE 2

Following the procedure and techniques of Example 1, 20 ml of 50% $H_2O_2$ (0.35 moles) were added to 200 grams of 80% $H_2SO_4$ (1.63 moles) and the mixture was heated to 50°C. Ten grams of n-decylsuccinic anhydride (0.042 moles) were added and the reaction mixture was stirred for about 4 hours. Upon filtration, 10.5 grams of n-decylbutanediperoxoic acid were obtained (86.7% of theory). A sample of this product showed 10.42% active oxygen (94% of theory).

EXAMPLE 3

Following the procedure and techniques of Example 1, 30 ml of 50% $H_2O_2$ (0.52 mole) were added to 225.5 grams of 88% sulfuric acid (2.02 moles). Ten grams of n-dodecylsuccinic anhydride (0.037 mole) were then added with stirring and the reaction mixture was stirred for about 4 hours at 55° - 60°C. A sample of dodecylbutanediperoxoic acid recovered showed 7.84% active oxygen (78% of theory).

The unexpected results demonstrated by Examples 1 through 3 are shown by Example 4 wherein decylsuccinic anhydride was added to concentrated sulfuric acid prior to contact with hydrogen peroxide.

EXAMPLE 4

Ten grams of decylsuccinic anhydride were mixed with 200 grams of 80% $H_2SO_4$ in a 250 ml beaker with stirring and heated to 50°C. After a few minutes a dense precipitate of decylsuccinic acid began

to appear. Heating was continued for an additional ten minutes to substantially complete the hydrolysis of the anhydride. Twenty ml of 50% $H_2O_2$ were added drop-wise to the mixture with stirring at 50°C and the mixture was allowed to react for about 4 hours. The reaction mixture was then filtered, washed and analyzed by iodometric titration as in Example 1. It showed an active oxygen content of less than 2%.

The following example illustrates the present process carried out in a continuous manner.

EXAMPLE 5

A solution was prepared by adding 900 ml of 50% $H_2O_2$ (15.9 moles) to a stirred vessel containing 6000 ml of 88% $H_2SO_4$ (89 moles) maintained at 20°C. The solution contained about 15% water. This solution was fed to an open 2 liter stirred reactor, fitted with temperature control means, at the rate of about 1000 ml per hour while particulate octylsuccinic anhydride was added to the reactor at the rate of about 150 grams per hour in 12.5 grams shots every 5 minutes. This rate provided a reaction mass having 17.5 moles $H_2SO_4$ and 3.3 moles $H_2O_2$ per mole of the octylsuccinic anhydride. The temperature of the reaction mass was maintained at 40° - 45°C and the reaction mass was allowed to overflow to another similar 2 liter reactor maintained at the same temperature. This provided a total average residence time of about 4 hours in the reactors. Samples from the overflow of the second reactor were taken at 1/2 hour intervals quenched with ice, filtered with a sintered glass funnel, washed with water, dried and analyzed for active oxygen content. Three such samples showed values of 11.6%, 11.8% and 11.8% active oxygen which corresponds to 95% - 97% of theory for octylbutanediperoxoic acid.

The foregoing examples demonstrate the unexpected high yield and reaction rate obtained by

the present process for making substituted-butanediperoxoic acid by the reaction of substituted succinic anhydride with a mixture of concentrated sulfuric acid and hydrogen peroxide.

The substituted-butanediperoxoic acids prepared by the process of this invention are particularly useful as the active component in bleach compositions used in laundrying fabrics.

Such diperoxoic acids can be formulated with suitable stabilizers to prevent exothermic decomposition, such as materials which are capable of liberating moisture at a temperature below the decomposition temperature of the particular substituted-butanediperoxoic acid compound. A wide variety of suitable exotherm control materials can be used and include hydrated materials such as potassium aluminum sulfate dodecahydrate, magnesium sulfate heptahydrate, sodium aluminum sulfate dodecahydrate, magnesium ammonium sulfate hexahydrate, acids, such as boric acid, malic acid, maleic acid, succinic acid, substituted-succinic acids, azelaic acid, dodecanedioic acid, cyclohexane dicarboxylic acid and the like. Boric acid is preferred.

Suitable stabilizers to prevent catalytic decomposition of the diperoxoic acids in the presence of heavy metals, for example, iron and copper, are chelating agents. Suitable chelating agents are alkali metal polyphosphates, ethylenediamine tetra acetic acid, 1-hydroxy-ethylidene diphosphonic acid, aminotri (methylene phosphonic acid), ethylenediaminetetra (methylenephosphonic acid), diethylenetriaminepenta (methylenephosphonic acid), phosphoric acid and mixtures thereof. Phosphoric acid or a mixture of phosphoric acid and tetrasodium pyrophosphate is preferred.

In addition to the chelating agents and exotherm control agents mentioned above, coating or encapsulation materials can also be used to extend the

shelf life of dry formulations containing the substituted-butanediperoxoic acids as the primary bleaching agent. Suitable coating materials include a wide variety of fatty acids, fatty alcohols, derivatives thereof, such as ethers and esters, derivatives of polyethylene glycols, such as esters and ethers, hydrocarbon oils, waxes and the like. These materials not only aid in preventing moisture from reaching the diperoxoic acid but can also be used to segregate the compound from other agents which may be present and adversely affect the diperoxoic acid's stability in the formulation. Other agents can include additional detergent materials such as surfactants, builders, anti-static agents, coloring agents, bleach activators, perfumes and the like.

A diluent can be employed as a processing aid to adjust the concentration of the diperoxoic acid as the primary bleaching agent in the formulation and to facilitate handling, shipping and subsequent addition to the wash water or blending with additional agents. The diluent or processing aid can be used in an amount to provide a formulation containing from about 30 to 60 percent by weight of the active substituted-butanediperoxoic acid, from about 1 to 5 percent by weight chelating agent, from about 15 to 55 percent by weight exotherm control agent. A preferred diluent is sodium sulfate which is compatible with the stabilizers as well as ingredients in detergent formulations.

The use of concentrated sulfuric acid as the reaction medium in the process of the present invention is compatible with and facilitates the processing of the substituted-butanediperoxoic acid into bleach formulations. For example, minor amounts of sulfuric acid retained in the filter cake of the product produced by the present invention can be neutralized with

a borax wash to provide some of the boric acid and sodium sulfate useful in producing the bleach formulations containing the substituted-butanediperoxoic acid product as the active agent.

Although the invention has been described in terms of specified embodiments which are set forth in considerable detail, it should be understood that this is by way of illustration only and that the invention is not necessarily limited thereto since alternative embodiments in operating techniques will become apparent to those skilled in the art in view of present disclosure. Accordingly, modifications are contemplated which can be made without departing from the spirit of the described invention.

1. Process for making substituted-butanediperoxoic acid which comprises reacting substituted succinic anhydride with a mixture of concentrated sulfuric acid and hydrogen peroxide.

2. The process of Claim 1 wherein the substituted succinic anhydride is of the general formula:

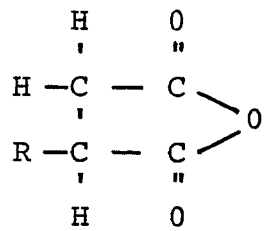

wherein R is alkyl of 6 to 18 carbon atoms or phenyl.

3. The process of Claim 2 wherein R is alkyl of 8 to 12 carbon atoms.

4. The process of Claim 1 wherein the mixture contains from about 10 to about 60 moles sulfuric acid and from about 2 to about 18 moles hydrogen peroxide per mole of substituted succinic anhydride.

5. The process of Claim 1 wherein the reaction is conducted at a temperature in the range of from about 15°C to about 60°C.

6. The process of Claim 1 wherein the mixture contains 20 to 40 moles sulfuric acid and 4 to 12 moles hydrogen peroxide per mole of substituted succinic anhydride and the reaction is conducted at a temperature in the range of about 25° to 55°C.

7. The process of Claim 6 wherein the substituted succinic anhydride is octylsuccinic anhydride.

8. The process of Claim 6 wherein the substituted succinic anhydride is dodecylsuccinic anhydride.

9. The process of Claim 6 wherein the substituted succinic anhydride is decylsuccinic anhydride.

10. The process of Claim 1 wherein the concentrated sulfuric acid is 75% to 98% $H_2SO_4$.

11. Continuous process for making a substituted-butanediperoxoic acid of the general formula:

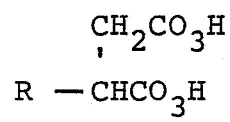

where R is alkyl of 6 to 18 carbon atoms or phenyl, which comprises continuously feeding to a stirred reaction system a mixture of concentrated sulfuric acid and hydrogen peroxide while feeding to said reactor system a substituted succinic anhydride of the formula:

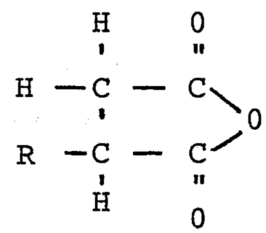

wherein R is as defined above and maintaining the feed rates of said mixture and substituted succinic anhydride to provide a reaction mass containing from about 10 to about 60 moles $H_2SO_4$ and from about 2 to about 18 moles $H_2O_2$ per mole of substituted succinic anhydride and an effective residence time in the reactor system to make said substituted-butanediperoxoic acid.

12. The process of Claim 11 wherein the reactor system is fitted with temperature control means and the reaction mass is maintained at a temperature in the range of about 15° to about 60°C.

13. The process of Claim 11 wherein R is alkyl of 8 to 12 carbon atoms.

14. The process of Claim 11 wherein the feed rates of said mixture and substituted succinic anhydride are maintained to provide a reaction mass containing 20 to 40 moles sulfuric acid and 4 to 12 moles hydrogen peroxide per mole of substituted succinic anhydride.

15. The process of Claim 14 wherein the feed rates are maintained to provide an effective residence

time in the reactor system in the range of about 30 minutes to 4 hours.

16. The process of Claim 14 wherein the reactor system is fitted with temperature control means and the reaction mass is maintained at a temperature in the range of about 25$^{o}$ to about 55$^{o}$C.

17. The process of Claim 16 wherein the concentrated sulfuric acid is 75% to 98% $H_2SO_4$.

18. The process of Claim 16 wherein the substituted succinic anhydride is octylsuccinic anhydride.

19. The process of Claim 16 wherein the substituted succinic anhydride is dodecylsuccinic anhydride.

20. The process of Claim 16 wherein the substituted succinic anhydride is decylsuccinic anhydride.